# EUROPÄISCHE PATENTSCHRIFT

(11) **EP 0 587 533 B1**
(45) Veröffentlichungstag und Bekanntmachung des Hinweises auf die Patenterteilung: **12.03.1997**
(21) Anmeldenummer: 93810624.2
(22) Anmeldetag: 31.08.1993
(51) Int. Cl.: C07C 229/08, C07C 221/00, C07C 231/10, C07C 209/50

(54) **Verfahren zur Herstellung von alpha-Aminoketonsalzen**
Process for the preparation of alpha-aminoketone salts
Procédé pour la préparation de sels d'alpha-aminocétones

(30) Priorität: 10.09.1992 CH 2848/92
(43) Veröffentlichungstag der Anmeldung: 16.03.1994
(73) Patentinhaber: Ciba Specialty Chemicals Holding Inc., 4057 Basel (CH)
(72) Erfinder: Drewes, Rolf Dr., D-64678 Lindenfels (DE); Friedrich, Hans-Helmut., D-64686 Lautertal 2 (DE)

(56) Entgegenhaltungen:
- DE-A- 4 215 159
- SYNTHETIC COMMUNICATIONS, 1972, NEW YORK Seiten 237 - 242 M. SUZUKI ET AL. 'convenient syntheses of aroylamino acids and alpha-amino ketones'
- PATENT ABSTRACTS OF JAPAN vol. 6, no. 180 (C-125) (1058) 14. September 1982 & JP-A-57 098 245 (FUJI ZOUKI SEIYAKU K.K.) 18. Juni 1982
- EUGEN MÜLLER 'Methoden der organischen Chemie' 1971 , GEORG THIEME VERLAG , STUTTGART Band X/1 * Seite 988 *

## Beschreibung

Die vorliegende Erfindung betrifft ein Verfahren zur Herstellung von Alpha-Aminoketonsalzen.

Alpha-Aminoketonsalze, insbesondere Phenacylaminhydrochlorid, finden z.B. als Edukte zur Synthese von Heterozyklen Verwendung. Als Stabilisatoren für PVC dienende Pyrrolverbindungen können beispielsweise mit Hilfe von Alpha-Aminoketonsalzen der Formel I hergestellt werden, wobei nach der bekannten Knorr'schen Pyrrol-Synthese vorgegangen werden kann [L. Knorr, H. Lange Ber. **35**, 2998 (1902)].

Verschiedene Verfahren zur Herstellung von Alpha-Aminoketonsalzen sind bekannt [s. z.B. M. Suzuki et al., Synth. Comm. **1972**, 237; S. Maeda et al., Chem. Pharm. Bull. **32**, 2536 (1984) C. Mannich, F. Hahn, Ber. **44**, 1542 (1911)]. Sie sind z.T. sehr umständlich (z.B. das Urotropin-Verfahren); erfordern Ausgangsmaterialien, die nicht kommerziell erhältlich sind oder sind technisch sehr aufwendig durchzuführen und damit wirtschaftlich wenig interessant.

Daher besteht weiterhin ein Bedarf nach einfachen und kostengünstigen Verfahren zur Herstellung dieser Produkte.

**Aus US 1,995,709 ist bekannt, aus Para- oder Meta-hydroxyphenoloximinoethylketon durch Reduktion an Pd-Kohle Verbindungen vom Typ** 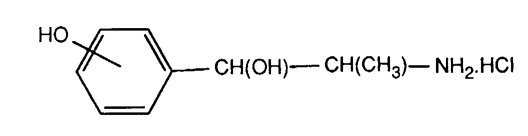 **herzustellen.**

Es wurde nun gefunden, daß sich alpha-Aminoketonsalze nach einem einfachen Verfahren herstellen lassen, das zweckmäßig auch ohne Isolierung der Zwischenprodukte durchgeführt werden kann.

Die Erfindung betrifft somit ein Verfahren zur Herstellung von alpha-Aminoketonsalzen der Formel I 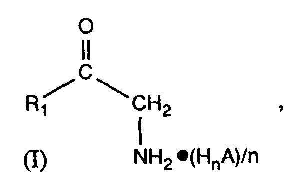 dadurch gekennzeichnet, daß ein nitrosierter Ketoester der Formel II 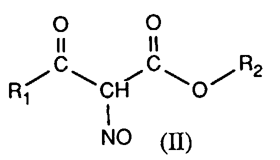 mit einem Carbonsäureanhydrid der Formel IV 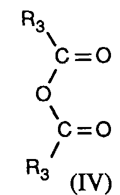 **unter heterogener katalytischer Hydrierung** zu einer Verbindung der Formel III 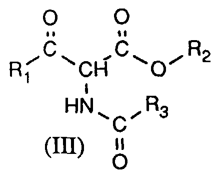 umgesetzt wird, welche dann mit einer Säure HₙA **bei 50 bis 120°C** zum Salz I hydrolysiert wird, wobei R₁ C₁-C₆-Alkyl, Phenoxy-C₁-C₄-alkyl, Phenyl, C₇-C₉-Phenylalkyl oder mit Halogen, C₁-C₆-Alkyl oder -Alkoxy, Hydroxy oder Cyano substituiertes Phenyl oder C₇-C₉-Phenylalkyl ist, R₂ C₁-C₄-Alkyl oder Cyclohexyl darstellt, n 1 bis 3 bedeutet, R₃ C₁-C₄-Alkyl und A der Rest einer organischen oder mineralischen Protonensäure ist.

R₁ als C₁-C₆-Alkyl bedeutet z.B. Methyl, Ethyl, Propyl, Isopropyl, n-Butyl, Isobutyl, t-Butyl, Pentyl, Isopentyl, Hexyl, 2-Ethylbutyl, 1-Methylpentyl oder 1,3-Dimethylbutyl.

R₂ und R₃ als C₁-C₄-Alkyl haben z.B. die ersten sieben der o.g. Bedeutungen.

Unter C₇-C₉-Phenylalkyl ist z.B. Benzyl, Phenethyl, α-Methylbenzyl oder α,α-Dimethylbenzyl zu verstehen.

C₁-C₆-Alkoxy bedeutet z.B. Methoxy, Ethoxy, Isopropoxy oder Hexoxy, bevorzugt Methoxy oder Ethoxy.

Phenoxy-C₁-C₄-alkyl bedeutet z.B. Phenoxymethyl, Phenoxyethyl oder Phenoxypropyl.

Substituiertes Phenyl oder Phenylalkyl kann einen oder mehrere, gleiche oder verschiedenen Substituenten tragen, z.B. 1 bis 3, bevorzugt einen oder zwei.

Als Carbonsäureanhydride der Formel IV kommen z.B. in Frage: Acetanhydrid, Propionsäureanhydrid, und Butter- oder Isobuttersäureanhydrid. Bevorzugt ist Acetanhydrid.

Denkbare Katalysatoren **für die erste Stufe des Verfahrens** sind z.B.: Raney Nickel oder Raney Kobalt sowie bevorzugt Edelmetallkatalysatoren auf Trägern, wie sie üblicherweise für katalytische Hydrierungen verwendet werden, insbesondere Pd/C, Pt/C, Ru/C, Pd-Pt/C-Mischkatalysatoren, Rh auf Al₂O₃; [...]
Besonders bevorzugt ist die heterogene Katalyse an Palladium, insbesondere an auf Kohlenstoffträger aufgebrachtem Palladium.

Die Dosierung des Katalysators liegt zweckmäßig bei 0.05 bis 0.5 Gew% Edelmetall bezogen auf die Verbindungen der Formel II, bevorzugt bei 0.1 bis 0.5%. Das Verhältnis Edelmetall : Träger liegt für gewöhnlich zwischen 1 und 10%.

Die Reaktanden der Formeln II und IV sowie der Wasserstoff werden zweckmäßig in ungefähr aequimolaren Mengen eingesetzt.

Die Hydrierung findet vorzugsweise unter Normaldruck und bei Temperaturen von beispielsweise 0 - 100°C, vorzugsweise 40 - 80°C, statt. Das Arbeiten bei erhöhtem Druck, beispielsweise zwischen 0 und 50 bar, vorzugsweise 0 und 10 bar, kann zweckmäßig sein.

Übliche Lösungsmittel wie z.B. Tetrahydrofuran, Diethylenglykoldimethylether, Essigsäureethylester oder Eisessig, vorzugsweise aber Eisessig, können bei der Hydrierung zugesetzt werden. Vorzugsweise wird der Reaktand IV als Lösungsmittel verwendet.

Es wurde überraschenderweise festgestellt, daß bei der sauren Verseifung des Amides der Formel III eine Verseifung der Estergruppe mit anschließender Decarboxylierung zum alpha-Aminoketon stattfindet. Die erwartete Reaktion zum Aminosäureester V 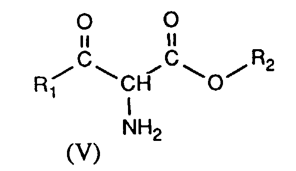 tritt nicht ein.

Die Verseifung im erfindungsgemäßen Verfahren (2. Stufe) erfolgt vorzugsweise bei 60 bis 100°C. Als Lösungsmittel dienen etwa Wasser bzw. die verdünnte Säure, Alkohole wie Ethanol, Propanol, Isopropanol, Ethylenglykol, Ethylenglykolether oder Ether wie Ethylenglykoldiethylether, Dioxan, Tetrahydrofuran.

Das Verfahren hat den Vorteil, daß eine Isolierung der Zwischenprodukte nicht erforderlich ist. Vorteilhaft für diese Ausführungsart ist die heterogen katalysierte Hydrierung.

Bevorzugt werden mit dem erfindungsgemäßen Verfahren Verbindungen der Formel I hergestellt, worin R₁ C₁-C₄-Alkyl, Phenyl, C₇-C₉-Phenylalkyl oder mit C₁-C₄-Alkyl substituiertes Phenyl- oder C₇-C₉-Phenylalkyl bedeutet, sowie solche, worin R₂ Methyl oder Ethyl und R₃ Methyl bedeuten.

Die Säure HA kann eine Mineral- oder organische Protonensäure sein. Beispiele sind HBr, HCI, H₂SO₄, H₃PO₄ und HCOOH, bevorzugt ist Salz- oder verdünnte Schwefelsäure. Entsprechend dient das Verfahren besonders bevorzugt der Herstellung von Phenacylaminhydrochlorid und Phenacylaminsulfat, d.h. HA steht in Formel I für HCl bzw. 1/2 H₂SO₄ und R¹ für Phenyl.

Die Verbindungen der Formel I sind bekannt und können, wie eingangs erwähnt [vgl. auch A. Alberola et al. Heterocycles **29**, 1973 (1989)], zu heterozyklischen Verbindungen für eine Reihe von Anwendungen, beispielsweise zu Pyrrolen, wie sie in EP-A-0 390 739 und 0 465 405 beschrieben sind, umgesetzt werden. Ebenfalls bekannt ist, daß die Ver bindungen der Formel I der Keto-Enol Tautomerie unterliegen:

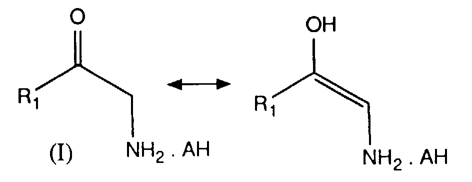

Das erfindungsgemäße Verfahren umfaßt die Herstellung beider Isomere, wenn auch der Einfachheit halber die Formel I nur die Ketoform zeigt.

Die Ausgangsstoffe der Formel II können aus kommerziell oder nach an sich bekannten Verfahren erhältlichen Acylessigsäureestern durch die ebenfalls bekannte Umsetzung mit NaNO₂ in Essigsäure hergestellt werden:

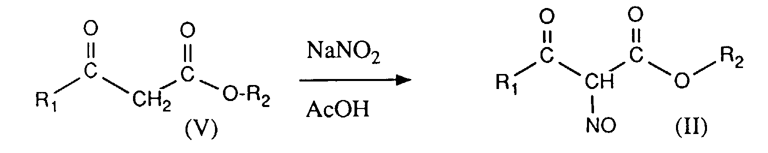

Die Vorstufe (Formel V, R₁ = Phenyl, R₂ = Ethyl) des in Beispiel 1 eingesetzten 2-Oximino-benzoylessigsäureethylesters ist kommerziell erhältlich.
Die folgenden Beispiele erläutern die Erfindung näher, ohne sie jedoch zu beschränken.
Teile- und Prozentangaben beziehen sich, sofern nicht anders angegeben, auf das Gewicht.

### Beispiel 1:

2-Acetamido-3-oxo-3-phenylpropionsäureethylester 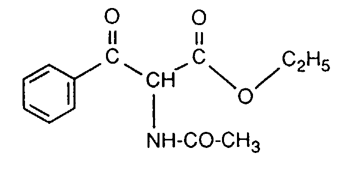 Ein Gemisch aus 221 g 2-Oximino-benzoylessigsäureethylester (2-Oximino-3-oxo-3-phenylpropionsäureethylester), 500 g Essigsäureanhydrid, 50 g Eisessig und 20 g eines **Pd/C** Katalysators (EF 101 R/W 51% H₂O der Firma Degussa) wird unter Normaldruck bei 60°C innerhalb 4 Stunden unter Rühren hydriert. Die Wasserstoffaufnahme beträgt 40 l. Anschließend wird das Reaktionsgefäß mit N₂ inertisiert, mit Stickstoff gespült und der Katalysator abfiltriert. Das Filtrat wird anschließend unter vermindertem Druck am Rotavapor auf Rückstand eingeengt (der Rückstand beträgt 259.3 g) und aus 200 ml Isopropanol/500 ml Petrolether umkristallisiert.

Ausbeute 175.4 g (70.4% d.Th.) weiße Kristalle, Smp. 81-82°C. Durch Abkühlen der Mutterlauge werden weitere 34.5 g (13.8%d.Th. Smp: 63-64°C; laut ¹H-NMR Gemisch aus Keto- und Enolform) isoliert.

Gesamtausbeute: 84.2%.

### Beispiel 2:

Phenacylamin Hydrochlorid 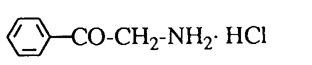 Die 259.3 g des Rohproduktes aus Beispiel 1 werden mit 375 g konzentrierter Salzsäure und 1000 ml Wasser 3 Stunden unter Rühren am Rückfluß erwärmt. Nach beendeter Gasentwicklung wird das Gemisch abgekühlt, die Trübung abfiltriert und das Filtrat am Rotavapor auf Rückstand eingeengt. Der Rückstand wird mit 250 ml Isopropanol/250 ml tert-Butyl-methylether aufgerührt, auf 10°C abgekühlt, der Niederschlag abgesaugt, mit tert-Butyl-methylether gewaschen und getrocknet.

Ausbeute: 132.7 g (77.5% d.Th.), berechnet pro in Beispiel 1 eingesetztem 2-Oximino-benzoylessigsäureethylester, weiße Kristalle, Smp: 187-88°C (Zersetzung).

### Beispiel 3:

Phenacylamin Hydrochlorid 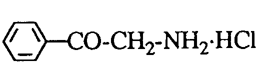 Wird statt des Rohprodukts der umkristallisierte 2-Acetamido-3-oxo-3-phenylpropionsäureethylester aus Beispiel 1 eingesetzt und in Analogie zu Beispiel 2 umgesetzt, so beträgt die Ausbeute 92.8% d.Th., Smp 189-91°C (Zersetzung).

### Beispiel 4:

Phenacylaminsulfat 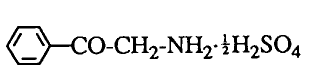 24.9 g 2-Acetamido-3-oxo-3-phenylpropionsäureethylester, 7.2 g Schwefelsäure (96 %ig) und 50 ml Wasser werden 10 Stunden unter Rühren am Rückfluß erwärmt. Nach dem Abkühlen wird das Reaktionsgemisch filtriert und das Filtrat am Rotavapor auf Rückstand eingeengt. Anschließend wird der kristalline Rückstand mit 50 ml Isopropanol angeteigt, die Kristalle abgesaugt, mit Petrolether (50-70°C) gewaschen und getrocknet.

Ausbeute: 12g (65% d.Th.), weiße Kristalle, Smp. 152-155°C (Zersetzung)

### Beispiel 5:

1-Aminobenzylaceton Hydrochlorid 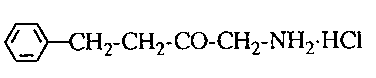 54 g 2-Acetamido-3-oxo-5-phenylvaleriansäuremethylester (60%ig), hergestellt analog Beispiel 1 aus 2-Oximino-3-oxo-5-phenylvaleriansäuremethylester, 79 g Salzsäure (32%ig) und 200 ml Wasser werden 2 h am Rückfluß gerührt. Anschließend wird das 2-phasige Reaktionsgemisch am Rotavapor auf Rückstand eingeengt. Der zum Teil kristalline Rückstand wird in 100 ml Isopropanol warm gelöst, mit 30 ml tert-Butyl-methylether versetzt, abgekühlt, und die ausgefallenen Kristalle werden isoliert.

Ausbeute 16.8 g (68% d.Th., ber. auf 100%igen 2-Acetamido-3-oxo-5-phenyl-valeriansäuremethylester) weiße Kristalle aus i-Propanol, Smp. 152°C (Zersetzung)

### Beispiel 6:

Aminoaceton Hydrochlorid: CH₃-CO-CH₂-NH₂·HCl

35 g 2-Acetamido-3-oxo-buttersäureethylester, hergestellt analog Beispiel 1 aus 2-Oximino-3-oxo-buttersäureethylester, 68 g Salzsäure (32%ig) und 180 ml Wasser werden 3 h bei 60 bis 70 °C gerührt. Nach beendeter CO₂-Entwicklung wird das Reaktionsgemisch auf Rückstand eingeengt.
Ausbeute: 20.3 g einer rotbraunen, viskosen Flüssigkeit, n_{D}³⁰: 1.488

## Patentansprüche

1. Verfahren zur Herstellung von alpha-Aminoketonsalzen der Formel I 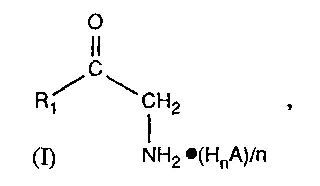 dadurch gekennzeichnet, daß ein nitrosierter Ketoester der Formel II 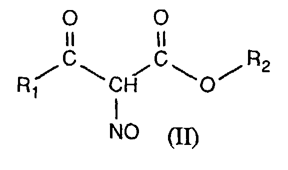 mit einem Carbonsäureanhydrid der Formel IV 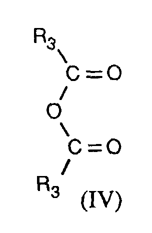 **unter heterogener katalytischer Hydrierung** zu einer Verbindung der Formel III 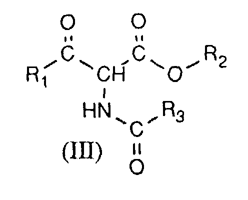 umgesetzt wird, welche dann mit einer Säure HₙA
**bei 50 bis 120°C**
zum Salz I hydrolysiert wird, wobei R₁ C₁-C₆-Alkyl, Phenoxy-C₁-C₄-alkyl, Phenyl, C₇-C₉-Phenylalkyl oder mit Halogen, C₁-C₆-Alkyl oder -Alkoxy, Hydroxy oder Cyano substituiertes Phenyl oder C₇-C₉-Phenylalkyl ist, R₂ C₁-C₄-Alkyl oder Cyclohexyl darstellt, n 1 bis 3 bedeutet, R₃ C₁-C₄-Alkyl und A der Rest einer organischen oder mineralischen Protonensäure ist.

2. Verfahren nach Anspruch 1, wobei R₁ C₁-C₄-Alkyl, Phenyl, C₇-C₉-Phenylalkyl oder mit C₁-C₄-Alkyl substituiertes Phenyl oder C₇-C₉-Phenylalkyl bedeutet.

3. Verfahren nach Anspruch 1, wobei R₂ Methyl oder Ethyl und R₃ Methyl bedeuten.

4. Verfahren nach Anspruch 1, dadurch gekennzeichnet, daß es ohne Isolierung von Zwischenprodukten durchgeführt wird.

5. Verfahren nach Anspruch 1, dadurch gekennzeichnet, daß die katalytische Hydrierung in Anwesenheit eines Palladium-Kohle-Katalysators durchgeführt wird.

6. Verfahren nach Anspruch 1, dadurch gekennzeichnet, daß für die Säure HₙA HBr, HCl, H₂SO₄, H₃PO₄ oder HCOOH, steht.

7. Verfahren nach Anspruch 1, dadurch gekennzeichnet, daß die Verbindung der Formel I Phenacylaminhydrochlorid oder Phenacylaminsulfat ist.

8. Verfahren nach Anspruch 1, dadurch gekennzeichnet, daß der Druck bei der Hydrierung bei 0 bis 50 bar liegt.

9. Verfahren nach Anspruch 1, dadurch gekennzeichnet, daß die Temperatur bei der Hydrierung bei 0 - **100°C** liegt.

## Claims

1. A process for the preparation of an alpha-amino ketone salt of formula I 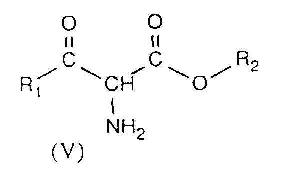 which comprises reacting a nitrosated keto ester of formula II 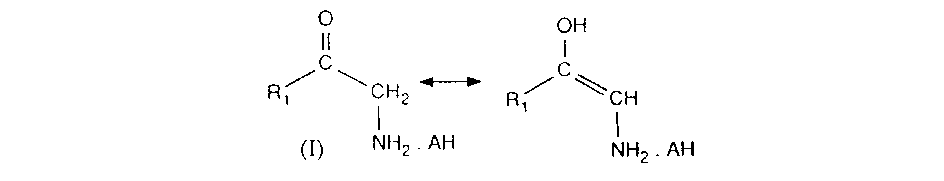 with a carboxylic anhydride of formula IV 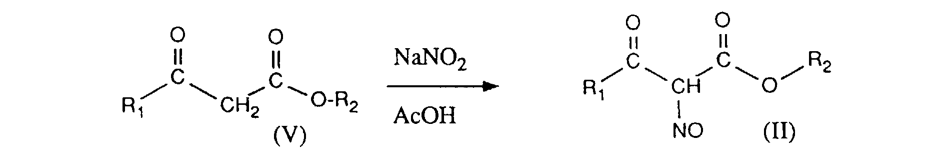 under conditions of heterogeneous catalytic hydrogenation to give a compound of formula III 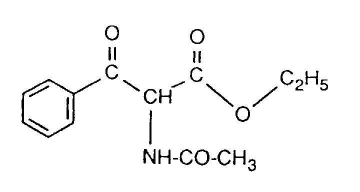 which is then hydrolysed at 50 to 120°C with an acid HₙA to give the salt I, where R₁ is C₁-C₆alkyl, phenoxy-C₁-C₄alkyl, phenyl, C₇-C₉phenylalkyl, or phenyl or C₇-C₉phenylalkyl which are substituted by halogen, C₁-C₆alkyl or C₁-C₆alkoxy, hydroxy or cyano, R₂ is C₁-C₄alkyl or cyclohexyl, n is 1 to 3, R₃ is C₁-C₄alkyl and A is the radical of an organic or mineral protic acid.

2. A process according to claim 1, wherein R₁ is C₁-C₄alkyl, phenyl, C₇-C₉phenylalkyl or C₁-C₄alkyl-substituted phenyl or C₇-C₉phenylalkyl.

3. A process according to claim 1, wherein R₂ is methyl or ethyl and R₃ is methyl.

4. A process according to claim 1, which is carried out without isolation of intermediates.

5. A process according to claim 1, wherein the catalytic hydrogenation is carried out in the presence of a palladium on carbon catalyst.

6. A process according to claim 1, wherein the acid HₙA is HBr, HCl, H₂SO₄, H₃PO₄ or HCOOH.

7. A process according to claim 1, wherein the compound of formula I is phenacylamine hydrochloride or phenacylamine sulfate.

8. A process according to claim 1, wherein the pressure during the hydrogenation is from 0 to 50 bar.

9. A process according to claim 1, wherein the temperature during the hydrogenation is from 0 to 100°C.

## Revendications

1. Procédé pour préparer des sels d'alpha-amino-cétones de formule I 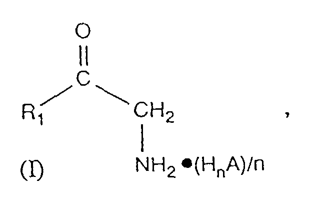 caractérisé en ce qu'on fait réagir un céto-ester nitrosé de formule II 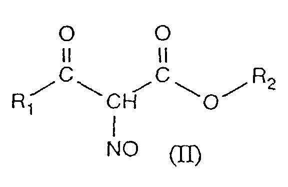 avec un anhydride carboxylique de formule IV 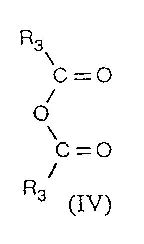 par hydrogénation catalytique hétérogène, pour donner un composé de formule III 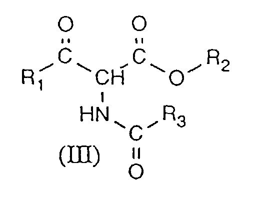 lequel est ensuite hydrolysé avec un acide HₙA, à une température de 50 à 120°C, pour donner le sel I, où R₁ est un radical alkyle en C₁-C₆, phénoxy(alkyle en C₁-C₄), phényle, phénylalkyle en C₇-C₉ ou encore phényle ou phénylalkyle en C₇-C₉ substitués par des substituants halogéno, alkyle ou alcoxy en C₁-C₆, hydroxy ou cyano, R₂ est un radical alkyle en C₁-C₄ ou cyclohexyle, n vaut de 1 à 3, R₃ est un radical alkyle en C₁-C₄, et A est le résidu d'un acide protonique organique ou minéral.

2. Procédé selon la revendication 1, dans lequel R₁ est un radical alkyle en C₁-C₄, phényle, phénylalkyle en C₇-C₉, ou encore phényle ou phénylalkyle en C₇-C₉ substitué par des substituants alkyle en C₁-C₄.

3. Procédé selon la revendication 1, dans lequel R₂ est le radical méthyle ou éthyle, et R₃ est le radical méthyle.

4. Procédé selon la revendication 1, caractérisé en ce qu'il est mis en oeuvre sans isolement de produits intermédiaires.

5. Procédé selon la revendication 1, caractérisé en ce que l'hydrogénation catalytique est mise en oeuvre en présence d'un catalyseur au palladium sur charbon.

6. Procédé selon la revendication 1, caractérisé en ce que, l'acide HₙA est représenté par HBr, HCl, H₂SO₄, H₃PO₄ ou HCOOH.

7. Procédé selon la revendication 1, caractérisé en ce que le composé de formule I est le chlorhydrate de phénacylamine ou le sulfate de phénacylamine.

8. Procédé selon la revendication 1, caractérisé en ce que la pression, lors de l'hydrogénation, est de 0 à 50 bar.

9. Procédé selon la revendication 1, caractérisé en ce que la température, lors de l'hydrogénation, est de 0 à 100°C.
